# EUROPEAN PATENT SPECIFICATION

(11) **EP 3 645 752 B1**
(45) Date of publication and mention of the grant of the patent: **21.02.2024**
(21) Application number: 18822684.9
(22) Date of filing: 01.06.2018
(51) Int. Cl.: C13K 1/02, C08H 8/00, C12P 19/00, C22C 38/08, B01J 19/00, C12P 7/10, D21C 1/04, D21C 3/04, C22C 38/02, C22C 38/44, C22C 38/58

(54) **APPARATUS AND METHOD FOR HYDROLYSIS OF LIGNOCELLULOSIC MATERIALS**
VORRICHTUNG UND VERFAHREN ZUR HYDROLYSE VON LIGNOZELLULOSESTOFFEN
APPAREIL ET PROCÉDÉ POUR L'HYDROLYSE DE MATIÈRES LIGNOCELLULOSIQUES

(30) Priority: 28.06.2017 SE 1750833
(43) Date of publication of application: 06.05.2020
(73) Proprietor: VALMET AB, 851 94 Sundsvall (SE)
(72) Inventor: SJÖBLOM, Krister, 856 34 Sundsvall (SE)
(74) Representative: Novagraaf Group
(86) International application number: PCT/SE2018/050563
(87) International publication number: WO 2019/004894

(56) References cited:
- EP-B1- 0 832 276
- EP-B1- 2 132 351
- WO-A2-00/78446
- WO-A2-2010/027323
- US-A1- 2008 318 291
- US-A1- 2010 056 774
- US-A1- 2012 156 748
- US-A1- 2012 156 748
- US-A1- 2016 076 067
- FERREIRA, E.A. et al.: "Evaluation of 316L Stainless Steel Corrosion Resistance in Solution Simulating the Acid Hydrolysis of Biomass", Journal of The Electrochemical Society, vol. 158, no. 4, 2011, pages C95-C103, XP055564249, ISSN: 0013-4651

## Description

### TECHNICAL FIELD

The present invention relates generally to a system and a method for feeding lignocellulosic material into an apparatus, e.g. a reactor vessel, in which saccharides are extracted by means of hydrolysis of the lignocellulosic material in the presence of a fluid, i.e. in an acidic water solution, and in particular to a system and a method for feeding a dewatered mixture of lignocellulosic material and fluid into an apparatus, wherein the moisture content of the dewatered mixture is measured before the dewatered mixture is introduced into the apparatus, and measures are taken to stop the feeding of the dewatered mixture into said apparatus if the measured moisture content is above a specified, predetermined level.

### BACKGROUND

Saccharides can be extracted from lignocellulose-containing materials, such as wood chips, sawdust and other biomass materials of wood and non-wood origin. The saccharides, e.g. pentoses or hexoses, can then be converted into liquid transportation fuels and bio-based platform and building-block chemicals. The process for extracting saccharides from a lignocellulosic material can include an acid-catalyzed hydrolysis reaction, in which the lignocellulosic material is mixed with an acidic water solution having a low pH value, e.g. in the interval of 0-4. The acidic water solution can, for example, be one with water highly diluted mineral acid, such as a diluted sulfuric acid. The hydrolysis is typically performed at high temperature, e.g. about or above 150 °C, and a challenge when performing the hydrolysis is that the combination of high temperature and low pH value makes the acidic water solution highly corrosive, which implies that apparatuses, such as reactor vessels, used in the hydrolysis process typically should be made from high-quality, corrosion-resistant materials, such as Ni-based alloys, tantalum, etc.

As is recognized in the European Patent No. 2132351 to van der Meulen et al., the presumed need for high-quality materials (and the considerable costs associated therewith) in the hydrolysis apparatuses used can hamper or even obstruct the industrial exploitation of the technologies and methods for extraction of saccharides from lignocellulosic materials. This European patent discloses, however, that relatively low-quality and thereby relatively inexpensive stainless-steel materials actually can be used in the apparatuses employed for the hydrolysis process. The suggested reason for this unexpected finding is that the lignocellulose-containing raw materials release one or more chemical substances that work as protective agents (protectors or inhibitors) for the stainless-steel materials used in the hydrolysis equipment. However, although this new finding represents a significant progress within the field, there are still problems to be solved. For example, since the industry typically wants to use different types of lignocellulosic materials (or the same lignocellulosic material but with varying characteristics, e.g. seasonal or geographical variations) as raw materials for the hydrolysis process, it is unsatisfactory that the preconditions for a problem-free use of the lignocellulosic raw materials together with the cheaper materials now suggested in the process apparatuses are not fully understood, not least since there are still considerable investments associated with, for instance, the start-up of a new process line for the extraction of saccharides from lignocellulosic materials. In other words, there is need for a method by which it is possible to determine when it is safe to introduce lignocellulosic materials in a hydrolysis apparatus, which at least partly is made from relatively inexpensive, low quality materials, i.e. when the properties of the lignocellulosic material are such that the apparatus does not corrode, or corrodes at an acceptable rate, and by which method the lignocellulosic material is prevented from entering the hydrolysis apparatus if it is determined that the properties of the lignocellulosic material are such that the apparatus will corrode at an unacceptable rate. A system configured for performing this a method is also needed.

An object of the present invention is therefore to provide an improved method for feeding lignocellulosic material into an apparatus in which extraction of saccharides from a lignocellulosic material is performed by hydrolysis of lignocellulosic material, which has been mixed with an acidic fluid, e.g. an acidic water solution, which method determines whether the mixture of fluid and lignocellulosic material fulfills the preconditions for successful use of specified steel materials in the apparatuses utilized for performing the hydrolysis reaction, i.e. the preconditions under which the steel materials in the apparatuses do not corrode, or do not corrode more or faster than an acceptable corrosion rate, and by which method the mixture of fluid and lignocellulosic material is prevented from entering the apparatuses if it is determined that the materials in the apparatuses will corrode more or faster than an acceptable rate. Another object is to, by applying the method according to the present invention, expand the set of steel materials that can be used in apparatuses for performing the hydrolysis reaction to preferably include steel materials having an even lower quality (and thereby being less expensive) than what previously have been deemed possible.

### SUMMARY OF THE INVENTION

The above-mentioned objects are achieved with a method and an apparatus according to the independent claims. Preferred embodiments are set forth in the dependent claims.

The invention is based on the finding that relatively inexpensive, low alloy steel materials can be used in hydrolysis apparatuses which come into contact with a mixture of lignocellulosic material and fluid as long as the mixture is dewatered to a moisture content below a certain predefined value, for example being dewatered to a moisture content below 70 %, which - in view of the suggestion that was brought forward in the aforementioned European Patent No. 2132351 that the non-corrosive conditions were created by protective agents released from the lignocellulosic materials - is a novel and surprising discovery. To exploit this new finding, a method according to the invention comprises measurement of the moisture content of a dewatered mixture of lignocellulosic material and fluid, comparison of the measured moisture content with a predefined value, e.g. 70 %, and an action to stop the feeding of the dewatered mixture into the hydrolysis apparatus if the moisture content is above the predefined value; and a system according to the invention comprises consequently means for carrying out such measurements, comparisons and stopping or preventing actions.

More specifically, a method for extraction of saccharides from lignocellulosic material comprises the steps of mixing the lignocellulosic material with a fluid (e.g. water, steam and/or an acid water solution), dewatering the mixture of the lignocellulosic material and the fluid, and introducing the dewatered mixture of the lignocellulosic material and the fluid into an apparatus and performing a hydrolysis in the apparatus, wherein at least parts of the apparatus that are configured for coming into contact with said dewatered mixture are made of a material that comprises iron in an amount of at least 50 % by weight and nickel in an amount of at least 1.5 % by weight. The method is characterized in that it further comprises the steps of measuring the moisture content of said dewatered mixture, comparing the measured moisture content with a predefined value and, if the measured moisture content is above said predefined value, preventing the dewatered mixture from entering the apparatus, wherein the hydrolysis is an acid-catalysed hydrolysis and the fluid is an acidic water solution. The dewatering of the mixture of lignocellulosic material and fluid can be accomplished with a plug screw feeder and the predefined value for the moisture content of the dewatered mixture can be about 70 %.

The invention also relates to a system for extraction of saccharides from lignocellulosic material, which system comprises a container configured for containing a lignocellulosic material, a source of fluid configured for adding a fluid to the lignocellulosic material, a dewatering device connected to the container and configured for dewatering a mixture of the lignocellulosic material and the fluid, and an apparatus connected to the dewatering device and configured for performing a hydrolysis of the dewatered mixture of the lignocellulosic material and the fluid, wherein at least parts of the apparatus that are configured for coming into contact with said dewatered mixture are made of a material that comprises iron in an amount of at least 50 % by weight and nickel in an amount of at least 1.5 % by weight. The system is characterized in that it further comprises means for measuring the moisture content of said dewatered mixture and means for comparing the measured moisture content with a predefined value and means for, if the measured moisture content is above said predefined value, preventing the dewatered mixture from entering the apparatus, wherein the fluid is an acidic water solution and the hydrolysis is an acid-catalysed hydrolysis. The container for containing the lignocellulosic material should be given a broad interpretation and can be any kind of vessel, tank, bin or receptacle or even a pipe or conduit. Similarly, the source of fluid can be any kind of vessel, tank, bin or receptacle or even a pipe or conduit, or a steam generator. The means for measuring the moisture content of the dewatered mixture of lignocellulosic material and fluid can comprise a sensor, whose operation, for example, is based on near-infrared technology, or capacity or conductance measurements. The comparison of the measured value for moisture content with a predefined value can be made in a computer or control unit, or can be done manually by an operator who reads the measured value from a display and compares the measured value with the predefined value. If a control unit is used, this unit is preferably connected to the dewatering device and is configured for controlling the operation of the dewatering device such that the control unit can stop the operation of the dewatering device and thereby preventing the mixture of lignocellulosic material and fluid from entering the hydrolysis apparatus. Alternatively, the control unit can be connected to a device, such as a valve, and configured for controlling the operation of the device, such that the mixture of lignocellulosic material and fluid is stopped from entering the hydrolysis apparatus by, for example, closing the valve.

As used herein, the terms "lignocellulose-containing material(s)" and "lignocellulosic material(s)" are used interchangeable and should be construed as having the same meaning. Further, apparatuses for extraction of saccharides from a lignocellulose-containing material comprise vessels, e.g. reactors, for performing the hydrolysis, but comprise also piping and feeding devices for transporting a slurry being a mixture of an acidic water solution and a lignocellulosic material. It should also be understood that it is not necessary that the whole apparatus is made from the specified material. Instead it is enough that the parts of the apparatus that come into contact with the slurry are made from a material comprising iron in an amount of at least 50 % by weight and nickel in an amount of more than 1.5 % by weight, which means, for example, that these parts can be coatings, layers or linings on or at the relevant surfaces of an apparatus made from another type of material.

### BRIEF DESCRIPTION OF THE DRAWINGS

The present invention will be further explained hereinafter by means of non-limiting examples and with reference to the appended drawings, wherein:

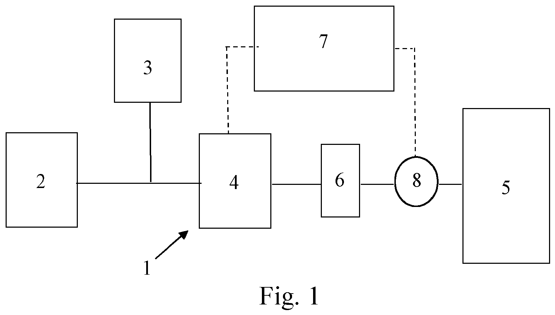

Fig. 1 is a schematic illustration of a system for performing extraction of saccharides from a lignocellulose-containing material according to the present invention.

### DETAILED DESCRIPTION

As was indicated above, a number of experimental results were disclosed in the aforementioned European Patent No. 2132351, from which results it was concluded that the rate of corrosion was acceptable for all materials tested except one; and that the rate of corrosion was surprisingly low also for the most inexpensive steel grade in the study. The speculation and the theory brought forward in EP2132351 to explain these findings are that since the acidic water solution, which in this case was a sulfuric acid solution, not only consists of sulfuric acid and water but also of smaller and greater amounts of different chemical substances that have been released from the lignocellulose-containing material, it is very likely that one or more of said released substances work as protective agent for the steel materials tested.

The theory about protective agents released from the lignocellulose-containing material was, however, challenged by the present inventor, and a method for extraction of saccharides from lignocellulosic material is suggested, which method comprises the following steps: mixing lignocellulosic material with a fluid; dewatering the mixture of lignocellulosic material and the fluid; and introducing the dewatered mixture of lignocellulosic material and the fluid into an apparatus and performing a hydrolysis in the apparatus, wherein at least parts of the apparatus that are configured for coming into contact with said dewatered mixture are made of a material that comprises iron in an amount of at least 50 % by weight and nickel in an amount of at least 1.5 % by weight. The method is further characterized in that it comprises the steps of measuring the moisture content of said dewatered mixture, comparing the measured moisture content with a predefined value and, if the measured moisture content is above said predefined value, preventing the dewatered mixture from entering the apparatus, wherein the hydrolysis is an acid-catalysed hydrolysis and the fluid is an acidic water solution. The pH value of the mixture of lignocellulosic material and fluid that is introduced into the apparatus can typically be in the interval of 0-4.

To test the method and to find a suitable predefined value for the moisture content, two experiments were done, the results of which are presented below. In these two examples, the steps of comparing the measured moisture content with a predefined value and, if the measured moisture content is above the predefined value, preventing the dewatered mixture from entering the apparatus were therefore not carried out.

### Example 1

Three different mixtures, being mixtures of sawdust and sulfuric acid, were produced with 85 % moisture content (mc), 70 % mc, and 50 % mc. The mixture sample (of a specific percentage of moisture content) was placed in a steel reactor vessel, which in turn was heated by a copper vessel, which stood insulated in an autoclave furnace, and corrosion probes were placed in the mixture sample. The corrosion probes, also referred to as electrodes, were made from five different alloys: 316L (EN 1.4404), SAF 2205 (EN 1.4462), SAF 2507 (EN 1.4401), 904L (EN 1.4539) and the Ni-based super-alloy 625 (EN 2.4856), the chemical compositions of which can be found in Table 1 below.

The corrosion measurements, which were performed at 200 °C, utilized so-called LPR (Linear Polarisation Resistance) monitoring, which involves measurement of the polarisation resistance of a corroding electrode using a small amplitude (~25 mV) sinusoidal polarisation of the electrode. The results, which are taken from graphs obtained during the study, are summarized in Table 2 below.

**Table 1: Chemical compositions [in weight-%] for the alloys used in Example 1 and Example 2.**

| Alloy | Fe | Cr | Ni | Mo | Mn | Si | P | S | N | C |
|---|---|---|---|---|---|---|---|---|---|---|
| 316L | Bal | 17 | 12 | 2.5 | 2 | 0.75 | 0.045 | 0.03 | 0.1 | 0.03 |
| SAF 2205 | Bal | 22 | 5 | 3.2 | < 2 | < 1 | <0.03 | <0.015 | 0.018 | 0.03 |
| SAF 2507 | Bal | 25 | 7 | 4 | 1.2 | 0.8 | 0.035 | 0.015 | 0.3 | 0.03 |
| 904 L | Bal | 21 | 25.5 | 4.5 | 2 | 1 | 0.45 | 0.035 | Cu:1.5 | |
| Alloy 625 | < 5 | 21.5 | Bal | 9 | 0.5 | 0.5 | 0.015 | 0.015 | Nb:0.1 | Al:0.4 |
| | | | | | | | | | Ta:4 | Co: 1 |
| LDX 2101 | Bal | 21.5 | 1.5 | 0.3 | 5 | | | | 0.22 | 0.03 |

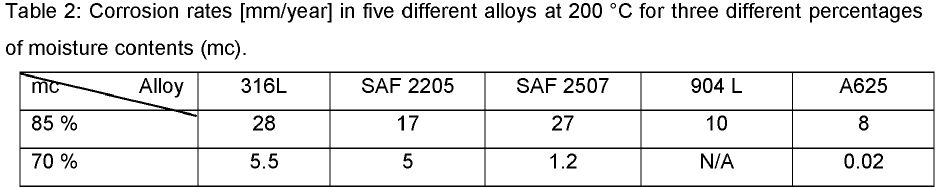

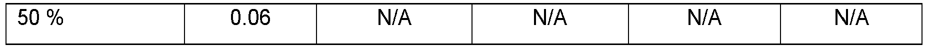

### Example 2

To verify the measurements done in Example 1, and to also investigate whether an even simpler steel grade can be used, the experiment above was repeated with LDX 2101 (EN 1.4162, see Table 1) and with SAF 2205 as reference sample. Three mixtures, being mixtures of sawdust and diluted sulfuric acid, were prepared with three different moisture contents: 85 % moisture content (mc), 70 % mc and 50 % me. The temperature was ramped up to 200 °C, where it was maintained. The test results are presented in Table 3 below.

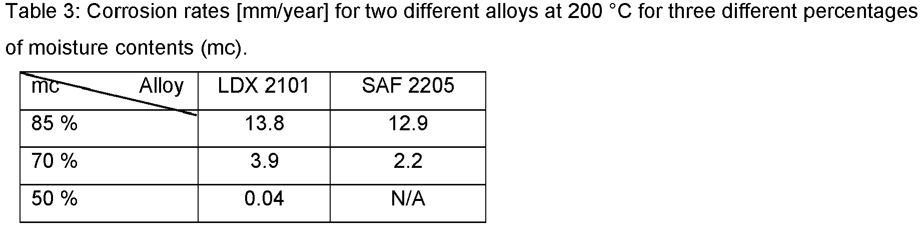

From Table 2 and Table 3 it is evident that the corrosion rates exhibit a clear dependence on the moisture contents of the mixtures, although a higher-quality steel grade generally corrodes less, as is expected. It can further be concluded that there is no linear dependence between the corrosion rate and the dry solid content of the mixture prepared, but instead it can be seen that for the lowest moisture content (i.e. a moisture content of 50 %) there is essentially no corrosion taking place even in the simplest steel grades, e.g. in LDX 2101 and 316L. (Generally, a steel grade having a corrosion rate below 0.1 mm/year is classified as corrosion resistant.) Further, also for a moisture content of 70 % in the mixture, the corrosion rates amount to a few millimeters per year also for the simplest steel grades, LDX 2101 and 316L, which - although presumably being too high for some applications - makes such relatively inexpensive steel grades interesting and useful as materials in apparatuses in, for example, pilot and test plants, which nevertheless are expected to have a relatively short useful life time, especially if the moisture contents of the mixtures introduced into these apparatuses actually are (at least somewhat) lower than 70 %. However, less expensive steel alloys having corrosion rates in the order of a few millimeters per year can be useful also in full-scale plants, especially if regular maintenance and replacement of spent parts can be accepted. It can further be noted that all the tested steel grades have an iron content of more than fifty percent. The simplest steel grade in the tests was LDX 2101, which is characterized by a nickel content of about 1.5 percent by weight, which therefore is considered to be the lowest useful nickel content for a material in an apparatus for hydrolysis of a mixture of a lignocellulosic material and an acidic water solution and having a moisture content of, preferably, at most about 70 %. In the examples, the hydrolysis is an acid-catalyzed hydrolysis, wherein the pH value of the mixture, for example, is within the interval of 0-4. The method according to the invention is, however, also applicable for an autohydrolysis wherein water or water vapor is added to the lignocellulosic material, to obtain a mixture having a moisture content which, preferably, is below 70 % and having a pH value in, for example, the interval of 0-4. Regarding the predefined value for a moisture content, it is however to be understood that a higher moisture content can be acceptable if an apparatus comprises or is made from a steel material having a higher quality; and it is also possible to measure the corrosion rate regularly and adjust the predefined value accordingly to an acceptable and suitable level, once the method is taken into practice.

A system 1 for carrying out the method according to the invention is schematically illustrated in Fig. 1, wherein the system 1 for extracting saccharides from a lignocellulose-containing material comprises a container 2 configured for containing lignocellulosic material; a source 3 of fluid configured for adding a fluid to the lignocellulosic material; a dewatering device 4 connected to the container 2 and configured for dewatering a mixture of lignocellulosic material and the fluid; and an apparatus 5 connected to the dewatering device 4 and configured for performing a hydrolysis of a dewatered mixture of lignocellulosic material and the fluid, wherein at least parts of the apparatus 5 that are arranged for coming into contact with said dewatered mixture are made of a material that comprises iron in an amount of at least 50 % by weight and nickel in an amount of at least 1.5 % by weight, wherein the fluid is an acidic water solution and the hydrolysis is an acid-catalysed hydrolysis. The system 1 comprises further means 6 for measuring the moisture content of said dewatered mixture and means 7 for comparing the measured moisture content with a predefined value, and means for, if the measured moisture content is above said predefined value, preventing the dewatered mixture from entering the apparatus 5, where said means for preventing the dewatered mixture from entering the apparatus 5 can be the dewatering device 4 or a blocking device 8, e.g. a valve 8.

The container 2 for the lignocellulosic material can have virtually any design, configuration and position, and the container 2 can, for example, be or comprise a receptacle, bin, tank or vessel for temporary storage, transport or delivery of the lignocellulosic material. It is also possible that the container 2 merely is or comprise a pipe or a conduit, such that there is no actual storage of the lignocellulosic material therein. Similarly, the source 3 of fluid can have virtually any design, configuration and position, and the source 3 of fluid can, for example, be or comprise a receptacle, bin, tank or vessel for temporary storage, transport or delivery of the fluid. It is also possible that source 3 of fluid merely is or comprise a pipe or a conduit, such that there is no actual storage of fluid therein; and when the fluid is water vapor, the source 3 of fluid can be a steam generator. The source 3 of fluid can be directly connected to the container 2, such that fluid is added to the lignocellulosic material contained in the container 2; or, as is depicted in Fig. 1, the source 3 of fluid can be arranged downstream of the container 2; or the source 3 of fluid can be arranged upstream of the container 2.

The dewatering device 4 can be a plug screw feeder, but also other dewatering devices, such as a press, are possible. The means 6 for measuring the moisture content of the dewatered mixture of lignocellulosic material and fluid comprises preferably a sensor 6, which, for example, can perform measurements of the moisture content of the dewatered mixture based on conductance or capacitance, or measurements based on near-infrared technology. Such sensors are generally known in the art.

The means 6 for measuring the moisture content of the dewatered mixture of fluid and lignocellulosic material is connected to means 7 for comparing a measured value of moisture content with a predefined value of moisture content, and the means 7 for comparing a measured value with a predefined value is preferably a computer or a control unit 7, in which the predefined value is stored and which is programmed for performing such a comparison. The connection between means 6 for measuring the moisture content and means 7 for comparing the measured moisture content with a predefined value can be a wired connection or a wireless connection.

In one embodiment of the invention, the means 7 for comparing a measured value with a predefined value, e.g. a control unit 7, is connected to the dewatering device 4, such that the control unit 7 controls the operation of the dewatering device 4, and the control unit 7 can in particular stop the operation of the dewatering device 4 if the measured moisture content is above the predefined value. When the dewatering device 4, e.g. a plug screw feeder 4, is taken out of operation, the transport of dewatered mixture of lignocellulosic material and fluid is stopped and is thereby prevented from entering the apparatus 5. In another embodiment of the invention, the control unit 7 can be operatively connected to a separate blocking device 8, which typically is a valve 8, such that the control unit 7 can close the valve 8 if the measured value for the moisture content of mixture of lignocellulosic material and fluid is above the predefined value.

The apparatus 5 for performing hydrolysis on the dewatered mixture of lignocellulosic material and fluid comprises typically a reactor vessel 5, but can further comprise also piping and feeding devices for transporting a dewatered mixture of lignocellulosic material and fluid. It should also be understood that it is not necessary that the whole apparatus is made from the specified material, i.e. a material having an iron content of more than about 50 % by weight and nickel in an amount of at least 1.5 % by weight. Instead it is enough that the parts of the apparatus that come into contact with the dewatered mixture are made from a material comprising iron in an amount of at least 50 % by weight and nickel in an amount of more than 1.5 % by weight, which means, for example, that these parts are coatings, layers or linings on or at the relevant surfaces of an apparatus made from another type of material.

Although the present invention has been described with reference to specific embodiments, it will be apparent to those skilled in the art that many variations and modifications can be done within the scope of the invention as described in the specification and defined with reference to the claims below.

## Claims

1. A system (1) for extraction of saccharides from lignocellulosic material, comprising:
a container (2) configured for containing lignocellulosic material;
a source (3) of fluid configured for adding a fluid to the lignocellulosic material;
a dewatering device (4) connected to the container (2) and configured for dewatering a mixture of lignocellulosic material and the fluid; and
an apparatus (5) connected to the dewatering device (4) and configured for performing a hydrolysis of a dewatered mixture of lignocellulosic material and the fluid, wherein at least parts of the apparatus (5) that are configured for coming into contact with said dewatered mixture are made of a material that comprises iron in an amount of at least 50 % by weight and nickel in an amount of at least 1.5 % by weight,
**characterized in that** the system (1) further comprises means (6) for measuring the moisture content of said dewatered mixture and means (7) for comparing the measured moisture content with a predefined value and means (4, 8) for, if the measured moisture content is above said predefined value, preventing the dewatered mixture from entering the apparatus (5), wherein the fluid is an acidic water solution and the hydrolysis is an acid-catalysed hydrolysis.

2. The system according to claim 1, **characterized in that** the predefined value is a moisture content of about 70 %.

3. The system according to any preceding claim, **characterized in that** the container (2) is a pipe, conduit, receptacle, bin, tank or vessel.

4. The system according to any preceding claim, **characterized in that** the source (3) of fluid is a pipe, conduit, receptacle, bin, tank, vessel or steam generator.

5. The system according to any preceding claim, **characterized in that** said means (6) for measuring the moisture content comprises a sensor (6).

6. The system according to any preceding claim, **characterized in that** said means (7) for comparing the moisture content with a predefined value comprises a control unit (7), which is connected to and configured for controlling the operation of the dewatering device (4).

7. The system according to any preceding claim, **characterized in that** said means (7) for comparing the moisture content with a predefined value comprises a control unit (7), which is connected to and configured for controlling the operation of a blocking device (8) which, when closed, prevents the dewatered mixture from entering the apparatus (5).

8. A method for extraction of saccharides from a lignocellulosic material, comprising the steps of:
mixing the lignocellulosic material with a fluid;
dewatering the mixture of the lignocellulosic material and the fluid; and
introducing the dewatered mixture of the lignocellulosic material and the fluid into an apparatus and performing a hydrolysis in the apparatus, wherein at least parts of the apparatus that are configured for coming into contact with said dewatered mixture are made of a material that comprises iron in an amount of at least 50 % by weight and nickel in an amount of at least 1.5 % by weight,
**characterized in that** the method further comprises the steps of measuring the moisture content of said dewatered mixture, comparing the measured moisture content with a predefined value and, if the measured moisture content is above said predefined value, preventing the dewatered mixture from entering the apparatus, wherein the fluid is an acidic water solution and the hydrolysis is an acid-catalysed hydrolysis.

9. The method according to claim 8, **characterized in that** the predefined value is a moisture content of about 70 %.

## Patentansprüche

1. System (1) für eine Extraktion von Sacchariden aus Lignocellulosematerial, umfassend:
einen Behälter (2), der zum Enthalten von Lignocellulosematerial konfiguriert ist;
eine Quelle (3) eines Fluids, die zum Hinzufügen eines Fluids zu dem Lignocellulosematerial konfiguriert ist;
eine Entwässerungsvorrichtung (4), die mit dem Behälter (2) verbunden ist und zum Entwässern einer Mischung aus Lignocellulosematerial und dem Fluid konfiguriert ist; und
eine Einrichtung (5), die mit der Entwässerungsvorrichtung (4) verbunden ist und zum Durchführen einer Hydrolyse einer entwässerten Mischung aus Lignocellulosematerial und dem Fluid konfiguriert ist, wobei mindestens Teile der Einrichtung (5), die zum in Kontakt kommen mit der entwässerten Mischung konfiguriert sind, aus einem Material hergestellt sind, das Eisen in einer Menge von zu mindestens 50 Gew.-% und Nickel in einer Menge von zu mindestens 1,5 Gew.-% umfasst,
**dadurch gekennzeichnet, dass** das System (1) ferner Mittel (6) zum Messen des Feuchtigkeitsgehalts der entwässerten Mischung und Mittel (7) zum Vergleichen des gemessenen Feuchtigkeitsgehalts mit einem vordefinierten Wert und Mittel (4, 8) zum Verhindern eines Eindringens der entwässerten Mischung in die Einrichtung (5), falls der gemessene Feuchtigkeitsgehalt über dem vordefinierten Wert liegt, umfasst, wobei das Fluid eine säurehaltige Wasserlösung ist und die Hydrolyse eine säurekatalysierte Hydrolyse ist.

2. System nach Anspruch 1, **dadurch gekennzeichnet, dass** der vordefinierte Wert ein Feuchtigkeitsgehalt von etwa 70 % ist.

3. System nach einem der vorstehenden Ansprüche,
**dadurch gekennzeichnet, dass** der Behälter (2) ein Rohr, eine Leitung, ein Auffanggefäß, ein Eimer, ein Becken oder ein Gefäß ist.

4. System nach einem der vorstehenden Ansprüche,
**dadurch gekennzeichnet, dass** die Quelle (3) des Fluids ein Rohr, eine Leitung, ein Auffanggefäß, ein Eimer, ein Becken, ein Gefäß oder ein Dampferzeuger ist.

5. System nach einem der vorstehenden Ansprüche,
**dadurch gekennzeichnet, dass** das Mittel (6) zum Messen des Feuchtigkeitsgehalts einen Sensor (6) umfasst.

6. System nach einem der vorstehenden Ansprüche,
**dadurch gekennzeichnet, dass** das Mittel (7) zum Vergleichen des Feuchtigkeitsgehalts mit einem vordefinierten Wert eine Steuereinheit (7) umfasst, die zum Steuern des Betriebs der Entwässerungsvorrichtung (4) verbunden und konfiguriert ist.

7. System nach einem der vorstehenden Ansprüche,
**dadurch gekennzeichnet, dass** das Mittel (7) zum Vergleichen des Feuchtigkeitsgehalts mit einem vordefinierten Wert eine Steuereinheit (7) umfasst, die zum Steuern des Betriebs einer Blockiervorrichtung (8) verbunden und konfiguriert ist, die, wenn sie geschlossen ist, verhindert, dass die entwässerte Mischung in die Einrichtung (5) eindringt.

8. Verfahren für die Extraktion von Sacchariden aus einem lignocellulosehaltigen Material, umfassend die Schritte:
Mischen des Lignocellulosematerials mit einem Fluid;
Entwässerung der Mischung des Lignocellulosematerials und des Fluids; und
Einführen der entwässerten Mischung des Lignocellulosematerials und des Fluids in eine Einrichtung und Durchführen einer Hydrolyse in der Einrichtung, wobei mindestens Teile der Einrichtung, die zum in Kontakt kommen mit der entwässerten Mischung konfiguriert sind, die aus einem Material hergestellt ist, das Eisen in einer Menge von zu mindestens 50 Gew.-% und Nickel in einer Menge von zu mindestens 1,5 Gew.-% umfasst,
**dadurch gekennzeichnet, dass** das Verfahren ferner die Schritte des Messens des Feuchtigkeitsgehalts der entwässerten Mischung, des Vergleichens des gemessenen Feuchtigkeitsgehalts mit einem vordefinierten Wert und des Verhinderns des Eindringens der entwässerten Mischung in die Einrichtung, falls der gemessene Feuchtigkeitsgehalt über dem vordefinierten Wert liegt, umfasst, wobei das Fluid eine säurehaltige Wasserlösung ist und die Hydrolyse eine säurekatalysierte Hydrolyse ist.

9. Verfahren nach Anspruch 8, **dadurch gekennzeichnet, dass** der vordefinierte Wert ein Feuchtigkeitsgehalt von etwa 70 % ist.

## Revendications

1. Système (1) d'extraction de saccharides à partir d'un matériau lignocellulosique, comprenant :
un récipient (2) conçu pour contenir un matériau lignocellulosique ;
une source (3) de fluide conçue pour ajouter un fluide au matériau lignocellulosique ;
un dispositif de déshydratation (4) relié au récipient (2) et configuré pour déshydrater un mélange de matière lignocellulosique et du fluide ; et
un appareil (5) relié au dispositif de déshydratation (4) et configuré pour effectuer une hydrolyse d'un mélange déshydraté de matière lignocellulosique et du fluide, dans lequel au moins des parties de l'appareil (5) qui sont conçues pour entrer en contact avec ledit mélange déshydraté sont constituées d'un matériau qui comprend du fer en une quantité d'au moins 50 % en poids et du nickel en une quantité d'au moins 1,5 % en poids,
**caractérisé en ce que** le système (1) comprend en outre un moyen (6) pour mesurer la teneur en humidité dudit mélange déshydraté et un moyen (7) pour comparer la teneur en humidité mesurée à une valeur prédéfinie et un moyen (4, 8) pour, si la teneur en humidité mesurée est supérieure à ladite valeur prédéfinie, empêcher le mélange déshydraté d'entrer dans l'appareil (5), dans lequel le fluide est une solution aqueuse acide et l'hydrolyse est une hydrolyse catalysée par un acide.

2. Système selon la revendication 1, **caractérisé en ce que** la valeur prédéfinie est une teneur en humidité d'environ 70 %.

3. Système selon une quelconque revendication précédente,
**caractérisé en ce que** le récipient (2) est un tuyau, un conduit, un réceptacle, un bac, un réservoir ou un récipient.

4. Système selon une quelconque revendication précédente,
**caractérisé en ce que** la source (3) de fluide est un tuyau, un conduit, un réceptacle, un bac, un réservoir, un récipient ou un générateur de vapeur.

5. Système selon une quelconque revendication précédente,
**caractérisé en ce que** ledit moyen (6) pour mesurer la teneur en humidité comprend un capteur (6).

6. Système selon une quelconque revendication précédente,
**caractérisé en ce que** ledit moyen (7) pour comparer la teneur en humidité à une valeur prédéfinie comprend une unité de commande (7), qui est connectée au dispositif de déshydratation (4) et configurée pour commander le fonctionnement de celui-ci.

7. Système selon une quelconque revendication précédente,
**caractérisé en ce que** ledit moyen (7) pour comparer la teneur en humidité à une valeur prédéfinie comprend une unité de commande (7), qui est connectée à un dispositif de blocage (8) et configurée pour commander le fonctionnement de celui-ci et, qui, lorsque le dispositif de blocage est fermé, empêche le mélange déshydraté de pénétrer dans l'appareil (5).

8. Procédé d'extraction de saccharides à partir d'un matériau contenant de la lignocellulose, comprenant les étapes consistant à :
mélanger la matière lignocellulosique avec un fluide ;
déshydrater le mélange de la matière lignocellulosique et du fluide ; et
introduire le mélange déshydraté de la matière lignocellulosique et du fluide dans un appareil et effectuer une hydrolyse dans l'appareil, dans lequel au moins des parties de l'appareil qui sont conçues pour entrer en contact avec ledit mélange déshydraté sont constituées d'un matériau qui comprend du fer en une quantité d'au moins 50 % en poids et du nickel en une quantité d'au moins 1,5 % en poids,
**caractérisé en ce que** le procédé comprend en outre les étapes consistant à mesurer la teneur en humidité dudit mélange déshydraté, comparer la teneur en humidité mesurée à une valeur prédéfinie et, si la teneur en humidité mesurée est supérieure à ladite valeur prédéfinie, empêcher le mélange déshydraté d'entrer dans l'appareil, dans lequel le fluide est une solution aqueuse acide et l'hydrolyse est une hydrolyse catalysée par un acide.

9. Procédé selon la revendication 8, **caractérisé en ce que** la valeur prédéfinie est une teneur en humidité d'environ 70 %.
